# EUROPÄISCHE PATENTSCHRIFT

(11) **EP 3 416 575 B1**
(45) Veröffentlichungstag und Bekanntmachung des Hinweises auf die Patenterteilung: **04.12.2019**
(21) Anmeldenummer: 16706788.3
(22) Anmeldetag: 19.02.2016
(51) Int. Cl.: A61B 17/80, A61B 17/86

(54) **VORRICHTUNG ZUR KNOCHENFIXATION**
DEVICE FOR BONE FIXATION
DISPOSITIF DE FIXATION OSSEUSE

(43) Veröffentlichungstag der Anmeldung: 26.12.2018
(73) Patentinhaber: 41medical AG, 2544 Bettlach (CH)
(72) Erfinder: FRIGG, Robert, 2544 Bettlach (CH); BURKI, Patrick, 3014 Bern (CH); FLURI, Daniel, 2544 Bettlach (CH)
(74) Vertreter: Lusuardi, Werther
(86) Internationale Anmeldenummer: PCT/CH2016/000032
(87) Internationale Veröffentlichungsnummer: WO 2017/139903

(56) Entgegenhaltungen:
- EP-A1- 2 801 330
- WO-A1-2004/049962
- US-A1- 2007 265 629
- US-A1- 2015 150 610
- US-B1- 8 784 458

## Beschreibung

Die Erfindung bezieht sich auf eine Vorrichtung zur Knochenfixation gemäss dem Oberbegriff des Patentanspruchs 1.

Eine Vorrichtung zur Knochenfixation mit den Merkmalen des Oberbegriffs des Anspruchs 1 ist aus der EP 2 801 330 A1 bekannt.

Der Erfindung liegt die Aufgabe zugrunde eine Vorrichtung zur Knochenfixation zu schaffen, welche eine sichere Verbindung zwischen dem Konstrukt Knochenschraube/Einsatz/Knochenplatte erlaubt und damit eine sichere Verankerung des Konstrukts als Ganzes am Knochen garantiert, wobei das Risiko des Verdrehens des Einsatzes in der Platte während des Verriegelungsvorgangs und des daraus resultierenden Ausreissens des zuvor geformten Gewindes im Knochen minimiert wird.

Die Erfindung löst die gestellte Aufgabe mit einer Vorrichtung zur Knochenfixation, welche die Merkmale gemäss dem Anspruch 1 aufweist. Vorteilhafte Weiterbildungen der Erfindung sind Gegenstand der abhängigen Ansprüche.

Die durch die Erfindung erreichten Vorteile sind im Wesentlichen darin zu sehen, dass dank der speziellen Materialpaarung bei der Einführung einer Knochenschraube mit Gewindekopf in das mit dem Einsatz versehene Plattenloch verdrängen die messerförmigen Flanken des Schraubenkopfgewindes, das - im Vergleich zum Knochenplattenmaterial relativ weiche - Material des Einsatzes, so dass ein fester Verbund zwischen Schraubenkopf - Einsatz - Knochenplatte erzielt wird.

Des Weiteren wird durch die verschiedenen Materialien ein Kaltverschweissen zwischen Schraube und Platte verhindert, was bei einer Revision der Platte von besonderer Relevanz ist.

Weitere vorteilhafte Ausgestaltungen der Erfindung können wie folgt kommentiert werden:
In einer speziellen Ausführungsform besteht die Knochenplatte aus einem Material der gleichen Härte wie das Material der Innenwand des Plattenlochs.

In einer anderen Ausführungsform liegt die Vickershärte des Materials des Einsatzes im Bereich von 20 bis 90% der Vickershärte des Materials der Knochenplatte.

In einer weiteren Ausführungsform liegt die Vickershärte des Materials des Einsatzes im Bereich von 120 bis 200HV.

In einer anderen Ausführungsform liegt die Vickershärte des Materials der Knochenplatte im Bereich 201 bis 600 HV.

In einer weiteren Ausführungsform bestehen die Knochenplatte und der Einsatz aus einem Metall oder einer Metalllegierung.

In einer weiteren Ausführungsform besteht die Knochenplatte aus einer Stahllegierung.

In wiederum einer weiteren Ausführungsform besteht die Knochenplatte aus Reintitan oder einer Titanlegierung.

In wiederum einer weiteren Ausführungsform besteht die Knochenplatte aus einer Cobalt-Chrom-Molybdän-Legierung.

In einer weiteren Ausführungsform besteht der Einsatz aus Reintitan, vorzugsweise aus Titan Grade 2.

In einer weiteren Ausführungsform ist das Plattenloch mit Mitteln versehen, welche die verdrehsichere Aufnahme des Einsatzes in der Plattenbohrung gestatten. Diese Ausführungsform kann z.B. durch einen oder mehrere Vorsprünge aus der Innenwand des Plattenloches und zu den Vorsprüngen entsprechenden Einkerbungen, Schlitzen oder durch den Einsatz durchgehenden Bohrungen realisiert werden. Ebenfalls kann die verdrehsichere Aufnahme des Einsatzes im Plattenloch durch die Vorsprünge an der Aussenwand des Einsatzes und dazu passenden Einkerbungen in der Innenwand des Plattenlochs realisiert werden.

In einer anderen Ausführungsform besitzt das Plattenloch eine unrunde oder zylindrisch unterbrochene Gestalt. Vorzugsweise hat das Plattenloch die Form eines Kreises mit mindestens einem fehlenden Kreissegment oder die Form von mindestens zwei sich teilweise überlappenden Kreisen.

In einer anderen Ausführungsform weist das Plattenloch eine elliptische Form auf.

In einer weiteren Ausführungsform besitzt das Plattenloch eine zentrale Achse, wobei der Einsatz konzentrisch zur zentralen Achse angeordnet ist. Der Vorteil dieser Ausführungsform ist darin zu sehen, dass ein konzentrisches Loch-Ring-Konstrukt eine erhöhte Winkelstabilität besitzt. Ein Ring der sich im Loch neigen kann lässt sich nicht so gut komprimieren, d.h. die Pressung zwischen Ring und Platte ist erniedrigt gegenüber einem konzentrischen Konstrukt.

In einer weiteren Ausführungsform ist die verdrehsichere Aufnahme des Einsatzes im Plattenloch mittels Formschluss realisiert. Dadurch ist der Einsatz gegen Verschieben, Schwenken und Verdrehen innerhalb des Plattenlochs gesichert.

In einer anderen Ausführungsform ist die verdrehsichere Aufnahme des Einsatzes im Plattenloch durch einen Anschlag im Plattenloch realisiert.

In einer anderen Ausführungsform weist der Einsatz einen durchgehenden Schlitz auf. Der Schlitz ermöglicht die Montage des Einsatzes in das Plattenloch durch elastische Verformung der unterbrochenen Geometrie.

In einer weiteren Ausführungsform weist der Einsatz einen Querschnitt in Form eines Kreises mit mindestens einem fehlenden Kreissegment auf.

In einer weiteren Ausführungsform weist der Einsatz einen Querschnitt mit im Wesentlichen der Form eines Polygons, vorzugsweise eines Dreiecks auf. In einer zusätzlichen Ausführungsform sind die Ecken des Polygons abgerundet.

In einer weiteren Ausführungsform ist der Einsatz fest mit der Knochenplatte verbunden.

In einer speziellen Ausführungsform ist die Knochenplatte (ohne Einsatz) einteilig ausgebildet.

In einer anderen Ausführungsform ist das Plattenloch zylindrisch ausgebildet.

In wiederum einer anderen Ausführungsform ist das Plattenloch konisch ausgebildet ist. Diese Ausführungsform ermöglicht Selbsthemmung des Einsatzes und des Plattenlochs.

In einer weiteren Ausführungsform liegt, der Halbkonuswinkel α des Plattenlochs im Bereich von 40° > α > 0° , vorzugsweise im Bereich 20° > α > 0° .

In einer anderen Ausführungsform ist der Einsatz im Plattenloch selbsthemmend angeordnet.

In einer anderen Ausführungsform ist der Einsatz im Plattenloch gegen axiale Verschiebung gesichert. Dies kann beispielsweise durch eine Materialausstülpung oder durch Schultern im Plattenloch erfolgen.

In einer weiteren Ausführungsform weist der Einsatz eine zum Plattenloch mindestens teilweise kongruente Gestalt auf.

In einer anderen Ausführungsform ist der Aussendurchmesser des Einsatzes grösser als der lichte Durchmesser des Plattenloches.

In einer weiteren Ausführungsform wird der Einsatz nicht durch Press-Fit im Plattenloch gehalten. Dadurch wird vermieden, dass die beiden Teile sich dekonnektieren können.

In einer speziellen Ausführungsform umfasst die Vorrichtung eine Knochenschraube, welche einen Kopf mit Aussengewinde aufweist.

In einer weiteren Ausführungsform ist das Aussengewinde des Schraubenkopfes ein mehrgängiges Gewinde.

In einer weiteren Ausführungsform liegt die Gewindesteigung des Kopfgewindes im Bereich zwischen 1 mm und 4 mm.

In einer anderen Ausführungsform ist das Aussengewinde des Schraubenkopfes ein eingängiges Gewinde.

In einer weiteren Ausführungsform liegt die Gewindesteigung des Kopfgewindes im Bereich zwischen 0,2 mm und 1 mm.

In einer weiteren Ausführungsform besitzt das Aussengewinde des Schraubenkopfes eine Gangtiefe von mindestens 0,1 mm. Der Vorteil dieser Ausführungsform ist darin zu sehen, dass die Gewindeflanken des Aussengewindes sich damit spanlos in den Einsatz durch Kaltumformung einschneiden können. Dadurch erhält das Einsatz-Platte-Konstrukt eine bessere Winkelstabilität, d.h. es ist fähig ein höheres Drehmoment im winkelstabilen Sinne aufzunehmen.

In einer anderen Ausführungsform verjüngt sich der Kopf der Knochenschraube mindesten teilweise in Richtung der Schraubenspitze.

In einer weiteren Ausführungsform besteht der Kopf der Knochenschraube aus einem Material, dessen Härte grösser als die Härte des Materials des Einsatzes ist. Dadurch wird die Verriegelung des Schraubenkopfes durch die Umformung des weicheren Einsatzes durch das härtere Material des Schraubenkopfes erreicht.

In einer anderen Ausführungsform besteht der Kopf der Knochenschraube aus einer Stahllegierung, z.B. Implantatenstahl 1.4441.

In einer anderen Ausführungsform besteht der Kopf der Knochenschraube aus Titan.

In einer weiteren Ausführungsform liegt die Vickershärte des Materials der Knochenschraube (20) im Bereich von 110 bis 500% der Vickershärte des Materials des Einsatzes.

In einer weiteren Ausführungsform sind die Vickershärte des Materials der Knochenschraube und die Vickershärte des Materials der Knochenplatte identisch. Vorzugsweise bestehen die Knochenschraube und die Knochenplatte aus dem gleichen Material.

In einer anderen Ausführungsform liegt die Vickershärte des Materials der Knochenschraube im Bereich 110 bis 500% der Vickershärte des Materials der Knochenplatte.

In einer weiteren Ausführungsform liegt die Vickershärte des Materials der Knochenschraube im Bereich von 201 bis 600HV.

Die Erfindung und Weiterbildungen der Erfindung werden im Folgenden anhand der teilweise schematischen Darstellungen mehrerer Ausführungsbeispiele noch näher erläutert.

Es zeigen:
Fig.1 eine perspektivische Ansicht einer Ausführungsform der erfindungsgemässen Vorrichtung;
Fig. 2 eine perspektivische Ansicht einer anderen Ausführungsform der erfindungsgemässen Vorrichtung;
Fig. 3 eine perspektivische Ansicht einer Ausführungsform des Plattenlochs der erfindungsgemässen Vorrichtung;
Fig. 4 eine perspektivische Ansicht einer anderen Ausführungsform des Plattenlochs der erfindungsgemässen Vorrichtung;
Fig. 5 eine schematische Ansicht einer weiteren Ausführungsform des erfindungsgemässen Einsatzes der erfindungsgemässen Vorrichtung;
Fig. 6 eine perspektivische Ansicht einer Ausführungsform des erfindungsgemässen Einsatzes;
Fig. 7 eine perspektivische Ansicht einer anderen Ausführungsform des erfindungsgemässen Einsatzes;
Fig. 8 eine Draufsicht einer Ausführungsform der Knochenschraube für die erfindungsgemässen Vorrichtung;
Fig. 9 einen Längsschnitt durch eine Ausführungsform einer erfindungsgemässen Vorrichtung;
Fig. 10 einen Längsschnitt durch eine andere Ausführungsform einer erfindungsgemässen Vorrichtung;
Fig. 11 einen Längsschnitt eines Einsatzes einer erfindungsgemässen Vorrichtung;
Fig. 12 eine Draufsicht eines Einsatzes einer erfindungsgemässen Vorrichtung;
Fig. 13 eine Ansicht einer Knochenplatte einer erfindungsgemässen Vorrichtung von unten;
Fig. 14 eine vergrösserte Ansicht der Fig. 13;
Fig. 15 einen Längsschnitt der Fig. 13;
Fig. 16 eine vergrösserte Ansicht der Fig. 15;
Fig. 17 einen Längsschnitt der Knochenplatte gemäss der Fig. 15 mit eingesetztem Einsatz;
Fig. 18 eine vergrösserte Ansicht der Fig. 17;
Fig.19 einen vergrösserte Längsschnitt einer erfindungsgemässe Vorrichtung mit einer eingesetzten Schraube und einem umgeformten Einsatz;

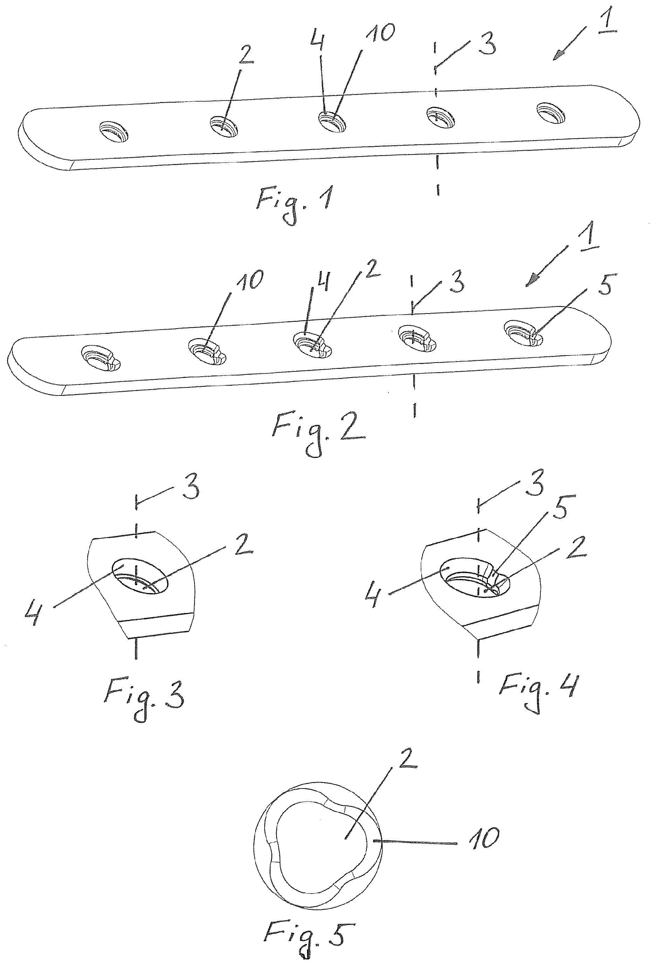

Die in Fig. 1 dargestellte Ausführungsform einer Vorrichtung zur Knochenfixation umfasst eine Knochenplatte 1 mit mehreren Plattenlöchern 2, welche je eine Zentralachse 3 und eine Innenwand 4 aufweisen. Das Plattenloch 2 ist zylindrisch ausgebildet und ist ausgestattet mit einem Einsatz 10, welcher zur Aufnahme eines Schraubenkopfes geeignet ist. Die Aussenwand des Einsatzes 10 ist kongruent zur Innenwand 4 des zylindrischen Plattenlochs 2 ausgebildet. Die verdrehsichere Anordnung des Einsatzes 10 im Plattenloch 2 erfolgt durch den Formschluss nach der Umformung der Platte über den Einsatz 10.

Die Fig. 1 zeigt einen zylindrischer Einsatz 10, welcher zuerst in die Platte eingelegt wird. Durch einen Stempel wird dann die Knochenplatte 1 unter Kraft verformt, so dass das Plattenmaterial über den Einsatz 10 fliesst. Durch die Freistellungen des Einsatzes 10 sind die Verdrehsicherung und die axiale Sicherung gegeben.

Die Einsätze 10 können ebenfalls konisch sein.

Die in Fig. 2 dargestellte Ausführungsform umfasst eine Knochenplatte 1 mit mehreren Plattenlöchern 2, welche je eine Zentralachse 3 und eine Innenwand 4 aufweisen. Das Plattenloch 2 weist eine unrunde bzw. zylindrisch unterbrochene Gestalt auf und ist mit einem in Richtung der Zentralachse 3 aus der Innenwand 4 herausragenden Vorsprung 5 ausgestattet. Das Plattenloch 2 umfasst einen Einsatz 10, welcher zur Aufnahme eines Schraubenkopfes geeignet ist. Der Einsatz 10 weist einen zum Vorsprung 5 passenden Schlitz 11 (die 11 ist in der Fig. 2 nicht eingezeichnet) auf, so dass der Einsatz 10 verdrehsicher im Plattenloch 2 angeordnet ist.

Fig. 3 zeigt eine perspektivische Ansicht auf einen Teil einer Knochenplatte 1 mit einem Plattenloch 2 mit einer Zentralachse 3 und einer Innenwand 4 ohne den eingesetzten Einsatz 10. Das Plattenloch 2 ist zylindrisch.

Fig. 4 zeigt eine perspektivische Ansicht auf einen Teil der Knochenplatte 1 mit einem Plattenloch 2 mit einer Zentralachse 3 und einer Innenwand 4 ohne den eingesetzten Einsatz 10. Das Plattenloch 2 weist eine unrunde bzw. zylindrisch unterbrochene Gestalt auf. Die Innenwand 4 des Plattenloches 2 ist versehen mit einem in Richtung der Zentralachse 3 herausragenden Vorsprung 5.

Fig. 5 zeigt eine schematische Ansicht eines Einsatzes 10, welcher eine Form eines Polygons (Dreiecks) mit abgerundeten Ecken aufweist.

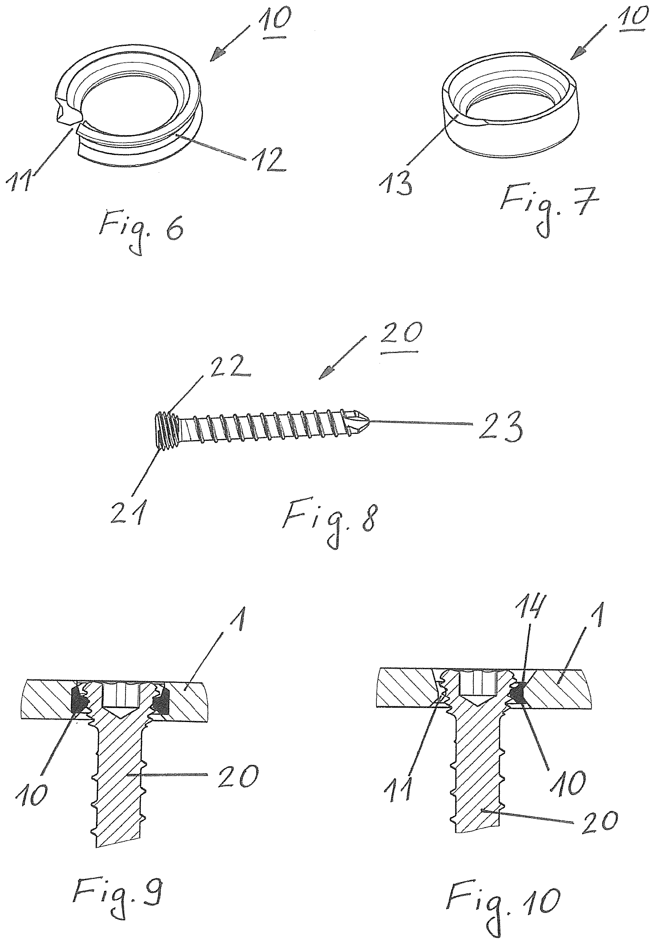

Fig. 6 zeigt eine perspektivische Ansicht eines Einsatzes 10 für eine Vorrichtung zur Knochenfixation. Der Einsatz 10 ist versehen mit einem durchgehenden Schlitz 11, mittels welchem die verdrehsichere Anordnung des Einsatzes 10 im Plattenloch 2 erreicht wird. Die Aussenwand des Einsatzes 10 weist eine radiale Furche 12 auf, welche beim Einsetzen des Einsatzes 10 in das Plattenloch 2 mit korrespondierenden Materialausstülpungen, eine Sicherung des Einsatzes 10 im Plattenloch 2 gegen axiale Verschiebung ermöglicht.

Fig. 7 zeigt eine perspektivische Ansicht eines anderen Einsatzes 10 für eine Vorrichtung zur Knochenfixation. Der Einsatz 10 ist versehen mit zwei Einkerbungen 13, welche - beim Einsetzen des Einsatzes 10 in das Plattenloch 2 mit korrespondierenden Schultern - nach dem Umformen (zum Beispiel durch Pressen, Schmieden oder Prägen) der Platte entstehen. Durch das Umformen und das entsprechenden Entstehen der zwei Einkerbungen wird sowohl eine Verdrehsicherung als auch eine Sicherung gegen axiale Verschiebung des Einsatzes 10 im Plattenloch 2 erzielt.

Fig. 8 ist eine Draufsicht einer Knochenschraube 20, welche einen mit Aussengewinde 22 versehenen Kopf 21 aufweist. Der Kopf 21 der Knochenschraube 20 verjüngt sich in Richtung der Schraubenspitze 23.

Fig. 9 zeigt einen Längsschnitt einer Knochenplatte 1 mit einem eingesetzten Einsatz 10 und einer Knochenschraube 20. Das Plattenloch 2 weist Einbuchtungen auf in welchen der Einsatz 10 positioniert ist, so dass der Einsatz 10 im Plattenloch 2 gegen axiale Verschiebung gesichert ist. Die Verriegelung des Schraubenkopfes 21 im Plattenloch 2 erfolgt durch die Umformung/Einformung des harten Schraubenkopfgewindes in das weichere Material des Einsatzes 10 (Titan) durch das härtere Material (Stahl) des Aussengewindes 22 des Kopfes 21 der Schraube 20. Der Einsatz 10 ist im Plattenloch 2 verdrehsicher angeordnet, so dass sich dieser während des Umformvorgangs nicht verdrehen kann.

Fig. 10 zeigt einen Längsschnitt einer weiteren Knochenplatte 1 mit einem eingesetzten Einsatz 10 und einer Knochenschraube 20. Der Einsatz 10 weist eine radiale Furche 12 (nicht gezeigt in Fig. 10) auf, welche mit der korrespondierenden Ausstülpung 14 des Plattenlochs 2 den Einsatz 10 im Plattenloch 2 gegen axiale Verschiebung sichert. Des Weiteren weist der Einsatz 10 einen durchgehenden Schlitz 11 auf. Fig. 10 zeigt den Längsschnitt einer Ausführungsform bei welcher der Längsschnitt durch den Schlitz 11 verläuft. Die Verriegelung des Schraubenkopfes 21 (nicht gezeigt in Fig. 10) im Plattenloch 2 erfolgt durch die Umformung des weicheren Materials des Einsatzes 10 (Titan) durch das härtere Material des Aussengewindes 22 (nicht gezeigt in Fig. 10) des Kopfes 21 der Schraube 20 (Stahl). Das Plattenloch 10 weist eine unrunde bzw. zylindrisch unterbrochene Gestalt auf, so dass der Einsatz 10 im Plattenloch 2 sich während des Einschraubvorgangs nicht verdrehen kann. Gleichzeitig mit der Umformung des Materials des Einsatzes 10 durch die Schraube 20 wird das weichere Material (Titan) des Einsatzes 10 in das härtere Material (Stahl) der Knochenplatte 1 eingedrückt. Der resultierende Form- und Kraftschluss sichert den Einsatz 10 rotativ und axial.

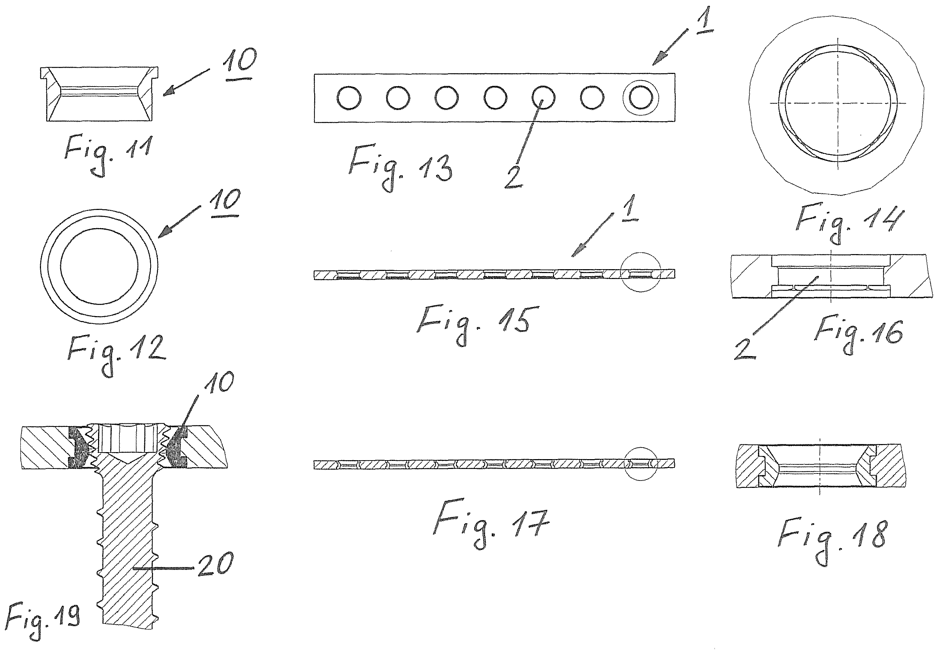

Fig. 11 zeigt einen zylindrischen Einsatz 10 im Schnittbild mit einer 2-fach konischen Innengeometrie und einer schmalen zylindrischen Rippe. Fig. 12 zeigt die Draufsicht des Einsatzes 10 mit einer Schulter. Fig. 13 zeigt eine Knochenplatte 1 mit den Plattenlöchern 2 in der Ansicht von unten. Fig. 14 ist eine Vergrösserung davon. Fig. 15 zeigt einen Längsschnitt der gleichen Knochenplatte 1 und Fig. 16 die Vergrösserung der Fig. 14. Fig. 16 zeigt ein Plattenloch 2 mit einem zylindrischen Profil mit 2 Schultern. Bei Fig. 16 ist diese oben tiefer als unten. Zusätzlich weist das Plattenloch 2 vier Freistellungen auf, welche nur auf einer Seite angebracht werden; diese dienen später der Verdrehsicherung.

### Vorgang:

Der Einsatz 10 wird von der Seite her, welche keine Freistellungen hat, in die Knochenplatte 1 eingepresst oder gelegt. Danach wird mit einem Stempel der untere Teil des Einsatzes 10 in die Schulter mit den Freistellungen verformt, zum Beispiel mit einer Presse. Das Material fliesst somit in die Freistellung und Schultern hinein. Der Formschluss, die Verdrehsicherung und die axiale Sicherung sind gegeben. Dies kann in Fig. 17 und Fig. 18 erkannt werden. Fig.19 zeigt eine eingeformte Knochenschraube mit dem umgeformten Einsatz 10 und der Knochenplatte 1.

Folgende Materialkombinationen sind besonders geeignet für die vorliegende Erfindung. Grade 2 und 4 bezeichnet Rein-Titan verschiedener Reinheitsgrade. Titan Grade 2 ist ein unlegiertes Titan mit einer Durchschnittshärte von 150HV (Vickershärte). Titan Grade 4 ist ein unlegiertes Titan mit erhöhtem Sauerstoffgehalt und einer Durchschnittshärte von 250HV (Vickershärte).

| Plattenmaterial | Einsatzmaterial | Schraubenmaterial |
|---|---|---|
| Titan Grade 4 | Titan Grade 2 | Titan Grade 4 |
| | | 1.4441 Implantatenstahl |
| Cobalt-Chrom-Molybdän | Titan Grade 2 | Titan Grade 4 |
| CCM | | 1.4441 Implantatenstahl |
| 1.4441 Implantatenstahl | Titan Grade 2 | Titan Grade 4 |
| | | 1.4441 Implantatenstahl |

## Patentansprüche

1. Vorrichtung zur Knochenfixation umfassend
a) eine Knochenplatte (1) mit einem Plattenloch (2) geeignet zur Aufnahme einer Knochenschraube (20), wobei das Plattenloch (2) eine Innenwand (4) aus einem Material der Härte H_{P} aufweist; sowie
b) einen im Plattenloch (2) gelagerten, mindestens teilweise an der Innenwand (4) anliegenden, hohlzylinder- oder hohlkegel-förmigen Einsatz (10) geeignet zur Aufnahme des Kopfes (21) der Knochenschraube (20), wobei
c) der Einsatz (10) im Plattenloch (2) verdrehsicher angeordnet und gegen axiale Verschiebung gesichert ist; und
d) der Einsatz (10) aus einem Material der Härte H_{E} < H_{P} besteht;
**dadurch gekennzeichnet, dass**
e) die verdrehsichere Aufnahme des Einsatzes (10) im Plattenloch (2) mittels Formschlusses realisiert ist, so dass der Einsatz gegen Verschieben, Schwenken und Verdrehen innerhalb des Plattenlochs gesichert ist.

2. Vorrichtung nach Anspruch 1, **dadurch gekennzeichnet, dass** die Knochenplatte (1) aus einem Material der gleichen Härte H_{P} besteht.

3. Vorrichtung nach Anspruch 1 oder 2, **dadurch gekennzeichnet, dass** die Vickershärte des Materials des Einsatzes (10) im Bereich von 20 bis 90% der Vickershärte des Materials der Knochenplatte (1) liegt.

4. Vorrichtung nach einem der Ansprüche 1 bis 3, **dadurch gekennzeichnet, dass** das Plattenloch (2) eine unrunde oder zylindrisch unterbrochene Gestalt besitzt.

5. Vorrichtung nach einem der Ansprüche 1 bis 4, **dadurch gekennzeichnet, dass** das Plattenloch (2) eine elliptische Form aufweist.

6. Vorrichtung nach einem der Ansprüche 1 bis 5, **dadurch gekennzeichnet, dass** das Plattenloch (2) eine zentrale Achse (3) besitzt und der Einsatz (10) konzentrisch zur zentralen Achse (3) angeordnet ist.

7. Vorrichtung nach einem der Ansprüche 1 bis 6, **dadurch gekennzeichnet, dass** die verdrehsichere Aufnahme des Einsatzes (10) im Plattenloch (2) durch einen Anschlag im Plattenloch realisiert ist.

8. Vorrichtung nach einem der Ansprüche 1 bis 7, **dadurch gekennzeichnet, dass** der Einsatz (10) einen durchgehenden Schlitz (11) aufweist.

9. Vorrichtung nach einem der Ansprüche 1 bis 8, **dadurch gekennzeichnet, dass** der Einsatz (10) einen Querschnitt in Form eines Kreises mit mindestens einem fehlenden Kreissegment aufweist.

10. Vorrichtung nach einem der Ansprüche 1 bis 9, **dadurch gekennzeichnet, dass** der Einsatz (10) einen Querschnitt mit im Wesentlichen der Form eines Polygons, vorzugsweise eines Dreiecks aufweist.

11. Vorrichtung nach einem der Ansprüche 1 bis 10, **dadurch gekennzeichnet, dass** der Einsatz (10) fest mit der Knochenplatte (1) verbunden ist.

12. Vorrichtung nach einem der Ansprüche 1 bis 11, **dadurch gekennzeichnet, dass** der Aussendurchmesser des Einsatzes (10) grösser als der lichte Durchmesser des Plattenloches (2) ist.

13. Vorrichtung nach einem der Ansprüche 1 bis 12, **dadurch gekennzeichnet, dass** sie eine Knochenschraube (20) umfasst, welche einen Kopf (21) mit Aussengewinde (22) aufweist.

14. Vorrichtung nach Anspruch 13, **dadurch gekennzeichnet, dass** der Kopf (21) der Knochenschraube (20) sich mindesten teilweise in Richtung der Schraubenspitze (23) verjüngt.

15. Vorrichtung nach Anspruch 13 oder 14, **dadurch gekennzeichnet, dass** der Kopf (21) der Knochenschraube (20) aus einem Material der Härte H_{S} > H_{E} besteht.

## Claims

1. A device for bone fixation, comprising:
a) a bone plate (1) with a plate hole (2) adapted to receive a bone screw (20), the plate hole (2) having an inner wall (4) made of a material with a hardness Hp; and
b) a hollow cylindrical or hollow conical insert (10) supported in the plate hole (2) and abutting at least partially the inner wall (4) and adapted to receive the head (21) of the bone screw (20), wherein
c) the insert (10) is arranged in the plate hole (2) in a rotation-proof manner and secured against axial displacement; and
d) the insert (10) consists of a material with the hardness H_{E} < H_{P};
**characterized in that**
e) the rotation-proof reception of the insert (10) in the plate hole (2) is realized by means of positive locking, so that the insert is secured against shifting, pivoting and rotating inside the plate hole.

2. The device according to claim 1, **characterized in that** the bone plate (1) consists of a material of the same hardness H_{P}.

3. The device according to claim 1 or 2, **characterized in that** the Vickers harness of the material of the insert (10) is in a range of 20 to 90% of the Vickers hardness of the material of the bone plate (1).

4. The device according to any one of claims 1 to 3, **characterized in that** the plate hole (2) has an out of round or cylindrically interrupted shape.

5. The device according to any one of claims 1 to 4, **characterized in that** the plate hole (2) has an elliptical shape.

6. The device according to any one of claims 1 to 5, **characterized in that** the plate hole (2) has a central axis (3) and the insert (10) is arranged concentrically to the central axis (3).

7. The device according to any one of claims 1 to 6, **characterized in that** the rotation-proof reception of the insert (10) in the plate hole (2) is realized by means of a stop in the plate hole.

8. The device according to any one of claims 1 to 7, **characterized in that** the insert (10) has a through slot (11).

9. The device according to any one of claims 1 to 8, **characterized in that** the insert (10) has a cross section in the form of a circle with at least one lacking circular segment.

10. The device according to any one of claims 1 to 9, **characterized in that** the insert (10) has a cross section substantially in the form of a polygon, preferably a triangle.

11. The device according to any one of claims 1 to 10, **characterized in that** the insert (10) is firmly connected to the bone plate (1).

12. The device according to any one of claims 1 to 11, **characterized in that** the outside diameter of the insert (10) is greater than the inside diameter of the plate hole (2).

13. The device according to any one of claims 1 to 12, **characterized in that** it comprises a bone screw (20) having a head (21) with an outside thread (22).

14. The device according to claim 13, **characterized in that** the head (21) of the bone screw (20) tapers at least partially in the direction of the screw tip (23).

15. The device according to claim 13 or 14, **characterized in that** the head (21) of the bone screw (20) consists of a material with the hardness H_{S} > H_{E}.

## Revendications

1. Dispositif de fixation osseuse, comprenant
a) une plaque d'os (1) dotée d'un trou de plaque (2) adapté à la réception d'une vis à os (20), dans lequel le trou de plaque (2) comporte une paroi interne (4) faite d'un matériau de dureté Hp ; et
b) un insert (10) disposé dans le trou de plaque (2), au moins partiellement adjacent à la paroi interne (4), en forme de cylindre creux ou de cône creux, adapté à la réception de la tête (21) de la vis à os (20), dans lequel
c) l'insert (10) est agencé dans le trou de plaque (2) sans possibilité de rotation ou coulissement axial ; et
d) l'insert (10) est constitué d'un matériau de dureté H_{E} < H_{P} ;
caractérisé en ce
e) la réception sans possibilité de rotation de l'insert (10) dans le trou de plaque (2) est réalisée au moyen d'une complémentarité de formes, de sorte que l'insert est assuré contre le coulissement, le pivotement et la rotation à l'intérieur du trou de plaque.

2. Dispositif selon la revendication 1, **caractérisé en ce que** la plaque d'os (1) est faite d'un matériau de la même dureté H_{P}.

3. Dispositif selon la revendication 1 ou 2, **caractérisé en ce que** la dureté Vickers du matériau de l'insert (10) est située dans un intervalle de 20 à 90 % de la dureté Vickers du matériau de la plaque d'os (1).

4. Dispositif selon l'une des revendications 1 à 3, **caractérisé en ce que** le trou de plaque (2) possède une configuration non-circulaire ou cylindrique interrompue.

5. Dispositif selon l'une des revendications 1 à 4, **caractérisé en ce que** le trou de plaque (2) possède une forme elliptique.

6. Dispositif selon l'une des revendications 1 à 5, **caractérisé en ce que** le trou de plaque (2) possède un axe central (3) et l'insert est agencé de manière concentrique à l'axe central (3).

7. Dispositif selon l'une des revendications 1 à 6, **caractérisé en ce que** la réception sans rotation de l'insert (10) dans le trou de plaque (2) est réalisée par une butée dans le trou de plaque.

8. Dispositif selon l'une des revendications 1 à 7, **caractérisé en ce que** l'insert (10) comporte une fente traversante (11).

9. Dispositif selon l'une des revendications 1 à 8, **caractérisé en ce que** l'insert (10) possède une section de forme circulaire avec au moins un arc de cercle manquant.

10. Dispositif selon l'une des revendications 1 à 9, **caractérisé en ce que** l'insert (10) possède une section de forme essentiellement polygonale, de préférence triangulaire.

11. Dispositif selon l'une des revendications 1 à 10, **caractérisé en ce que** l'insert (10) est attaché de manière fixe à la plaque d'os (1).

12. Dispositif selon l'une des revendications 1 à 11, **caractérisé en ce que** le diamètre extérieur de l'insert (10) est supérieur au diamètre intérieur du trou de plaque (2).

13. Dispositif selon l'une des revendications 1 à 12, **caractérisé en ce qu'**il comprend une vis à os (20), laquelle comporte une tête (21) dotée d'un filetage extérieur (22).

14. Dispositif selon la revendication 13 **caractérisé en ce que** la tête (21) de la vis à os (20) se rétrécit au moins partiellement dans la direction de la pointe de vis (23).

15. Dispositif selon la revendication 13 ou 14, **caractérisé en ce que** la tête (21) de la vis à os (20) est constituée d'un matériau de dureté H_{S} > H_{E}.
